# EUROPEAN PATENT APPLICATION

(11) **EP 3 871 771 A1**
(43) Date of publication of application: **01.09.2021**
(21) Application number: 21162633.8
(22) Date of filing: 18.08.2016
(51) Int. Cl.: B01J 20/24, B01D 15/38, B01J 20/28, B01J 20/286, B01J 20/32, C07K 1/22

(54) **CHROMATOGRAPHY SYSTEM ARRANGED FOR PERFORMING CONTINUOUS CHROMATOGRAPHY**

(30) Priority: 28.08.2015 GB 201515339
(62) Divisional of application: 16757842.6
(71) Applicant: Cytiva BioProcess R&D AB, 751 84 Uppsala (SE)
(72) Inventor: Skoglar, Helena, 751 84 Uppsala (SE); Lacki, Karol, 751 84 Uppsala (SE); Laurin, Linus, 751 84 Uppsala (SE); Blom, Hans, 751 84 Uppsala (SE)
(74) Representative: Démoulin, Eva Lotta

(57) **Abstract**

The invention discloses a chromatography system arranged for performing continuous chromatography, said system comprising at least two, such as at least three chromatography columns (11,12,13), connected with one or more connecting lines (14,15,16) such that liquid can flow from one column (11,12) to a subsequent one (12,13) and from a last column (13) to a first column (11) and each connecting line (14, 15, 16) between two columns may comprise at least one on/off valve (17,18,19), wherein each column is packed with a separation matrix comprising porous spherical particles to which antibody-binding protein ligands have been covalently immobilized, wherein the density of said ligands is above 10 mg/ml, and the volume-weighted median diameter of said particles is in the range of 30 - 55 μ m; and said porous spherical particles comprise a crosslinked polysaccharide and have a gel phase distribution coefficient, expressed as K _{D} for dextran of molecular weight 110 kDa, of 0.6-0.8.

## Description

### Technical field of the invention

The present invention relates to separation matrices, and more particularly to a separation matrix useful in antibody separation. The invention also relates to a method of separating antibodies on the matrix.

### Background of the invention

In the manufacturing of therapeutic monoclonal antibodies (mAbs), affinity chromatography on matrices comprising coupled *Staphylococcus* Protein A (SpA) or variants of SpA is commonly used as a first separation step to remove most of the contaminants. As the demand for therapeutic mAbs is increasing there is a strong driving force for improving the efficiencies of the separation processes and several approaches are under evaluation.

Multicolumn continuous chromatography processes are available, where the feed is applied to a first column and is then diverted to one or more subsequent columns as the first columns approaches saturation and the first column is eluted and regenerated to be loaded again during elution and regeneration of the subsequent column(s). Such processes can be denoted periodic countercurrent chromatography (PCC) or simulated moving bed (SMB) and are of considerable interest for separation of therapeutic mAbs, see e.g. US7901581, US20130248451, US20130280788 and US7220356, which are hereby incorporated by reference in their entireties. PCC/SMB processes can significantly increase the productivity, but it appears that the full potential cannot be reached with currently available separation matrices, which are designed for conventional batch chromatography.

Accordingly there is a need for new separation matrices specifically designed for continuous chromatography processes and for processes using such matrices.

### Summary of the invention

One aspect of the invention is to provide a separation matrix allowing continuous separation of mAbs with high productivity. This is achieved with a matrix as defined in claim 1.

One advantage is that the matrix has a high binding capacity at very short residence times.

A second aspect of the invention is to provide a chromatography column allowing continuous separation of mAbs with high productivity. This is achieved with a column as defined in the claims.

A third aspect of the invention is to provide a multicolumn chromatography system allowing continuous separation of mAbs with high productivity. This is achieved with a system as defined in the claims.

A fourth aspect of the invention is to provide an efficient method of separating antibodies. This is achieved with a method as defined in the claims. One advantage is that the method allows very short residence times with high binding capacity.

Further suitable embodiments of the invention are described in the dependent claims.

### Drawings

Fig. 1 shows an alignment of Protein A Fc-binding domains.
Fig. 2 shows a chromatogram from Example 1. UV Sample Pre = UV absorbance of the feed, UV Sample Post = UV absorbance of column effluent.
Fig. 3 shows the dynamic binding capacity for a matrix of the invention, compared with a reference matrix.
Fig. 4 shows a column according to the invention.
Fig. 5 shows a chromatography system according to the invention.

### Detailed description of embodiments

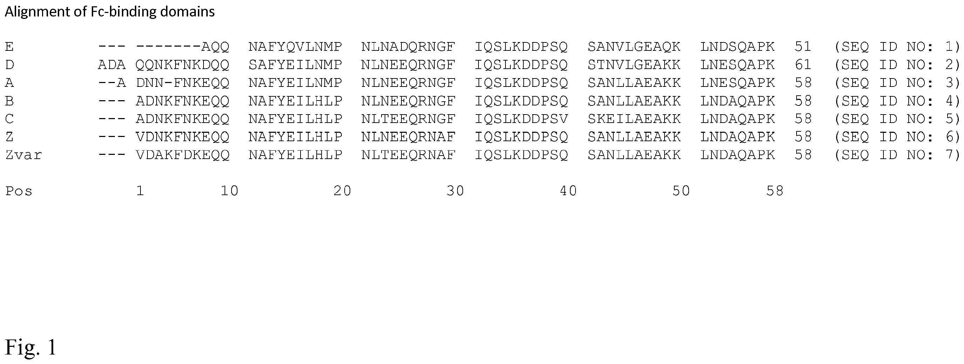

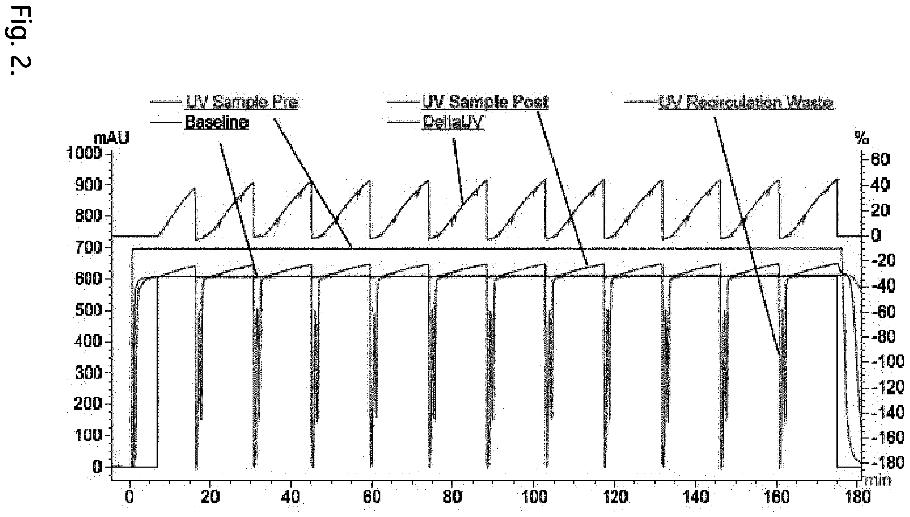

In one aspect, illustrated by Figs. 1-3, the present invention discloses a separation matrix comprising porous, suitably spherical, particles to which antibody-binding protein ligands have been covalently immobilized. The density of these ligands is above 10 mg/ml, e.g. in the range of 10.5 - 15 mg/ml, such as 10.5-12 mg/ml, and the volume-weighted median diameter of the particles is in the range of 30 - 55 µm, such as 45 - 55 µm or 50-55 µm. The density of the ligands denotes the content of coupled ligands per ml matrix sediment volume and it can be determined e.g. by amino acid analysis. The volume weighted median diameter, also denoted d50,v, can be determined by electrozone sensing (Coulter Counter), laser light diffraction or microscopy with image analysis. A preferred method is to use electrozone sensing with an instrument calibrated with a narrow sieve fraction of the matrix in question, for which the d50,v, has been determined with microscopy and image analysis.

The porous particles may comprise a crosslinked polysaccharide, which provides a large hydrophilic surface for coupling of the ligands, with minimal risk of non-specific interactions between mAbs or contaminants and the particles. The polysaccharide suitably has zero or very low (e.g. < 5 micromol/ml) content of charged groups to prevent non-specific interactions. The crosslinking increases rigidity and chemical stability and can be achieved by methods known in the art, in particular by epoxide crosslinking, using e.g. epichlorohydrin or a diepoxide as crosslinker. Examples of polysaccharides can be dextran, cellulose and agarose. Agarose has the particular advantage that highly porous, rigid gels can be achieved by thermal gelation of aqueous agarose solution. The agarose can suitably be crosslinked by the methods described in US6602990, US7396467 or US8309709, which are hereby incorporated by reference in their entireties. Agarose crosslinked by these methods, so called high flow agarose, has a particularly advantageous combination of high rigidity and high porosity/pore volume, allowing high flow rates and rapid mass transport. High rigidity is particularly important for matrices having small particle sizes, to allow high flow rates without collapse of the matrix. The agarose can e.g. be allylated with reagents like allyl glycidyl ether or allyl halides before gelation, as described in US6602990. To allow for high binding capacities and rapid mass transport, the particles can advantageously have a large volume of pores accessible to macromolecular species like IgG antibodies. This can be determined by inverse size exclusion chromatography (SEC) as described in "Handbook of Process Chromatography, A Guide to Optimization, Scale-Up and Validation" (1997) Academic Press, SanDiego, Gail Sofer & Lars Hagel eds. ISBN 0-12-654266-X, p. 368. A suitable parameter for the accessible pore volume is the gel phase distribution coefficient, K_{D}, determined for a probe molecule of defined size. This is a column-independent variable calculated from the retention volume V_{R} for the probe molecule, the interstitial void volume of the column V₀ and the total liquid volume of the column Vt according to K_{D} = (V_{R}-V₀)/(Vₜ-V₀). The porous particles can suitably have a K_{D} value in the range of 0.6-0.8, such as 0.65-0.75 or 0.65-0.70, for dextran of molecular weight 110 kDa as the probe molecule.

The ligands can e.g. be derived from antibody-binding bacterial proteins, such as SpA (Protein A), *Peptostreptococcus* Protein L or *Streptococcus* Protein G. They may bind to antibodies such that the K_{D} value of the interaction is at most 1 x 10⁻⁶ M, for example at most 1 x 10⁻⁷ M, such as at most 5 x 10⁻⁸ M. They can comprise an Fc-binding protein, such as SpA or and SpA variant, which binds to the Fc part of IgG molecules. They can comprise monomers, dimers or multimers of native or mutated Protein A Fc-binding domains. The native Protein A Fc-binding domains E, D, A, B and C are shown in Fig. 1, together with the mutated variants Z and Zvar. In some embodiments, one or more of the domains in the ligands is derived from Protein Z or the B or C domain of Protein A, with the amino acid residue at position 23 being a threonine. Such domains have an improved alkali stability desirable for bioprocess use (see e.g. US8329860, US7834158, US 14/961164 and WO2016079033, hereby incorporated by reference in their entireties), which may e.g. be assessed by incubating the separation matrix 5 h in 0.5 M NaOH at 22 +/- 2 °C. Suitably, the matrix then retains at least 90% or at least 95% of the original IgG-binding capacity before incubation. In certain embodiments, one or more of the domains comprises an amino acid sequence as defined by SEQ ID NO: 8 or 9. SEQ ID NO:8 is the Zvar domain minus the linker sequence VDAKFD and SEQ ID NO:9 is the C domain minus the linker sequence ADNKFN. One or more of the domains, such as all the domains, may also be mutated by one or more amino acid substitutions, insertions or deletions. Thus for example, there may be up to 10, 9, 8, 7, 6, 5, 4, 3 or 2 mutations, e.g. substitutions within SEQ ID NO: 8 or 9.
SEQ ID NO:8
   KEQQ NAFYEILHLP NLTEEQRNAF IQSLKDDPSQ SANLLAEAKK LNDAQAPK
SEQ ID NO:9
   KEQQ NAFYEILHLP NLTEEQRNGF IQSLKDDPSV SKEILAEAKK LNDAQAPK

The ligands may additionally comprise one or more linker sequences of 1-10 amino acid residues, e.g.VDNKFN, ADNKFN, VDAKFD, AD or FN, suitably between the individual domains. In addition, the ligands may comprise a coupling moiety, e.g. a cysteine or a plurality of lysines at the C-terminus or N-terminus of the ligand, suitably at the C-terminus. The ligands may also comprise a leader sequence at the N-terminus, e.g. a scar or a residue after cleavage of a signal peptide and optionally also a copy of a linker sequence. Such a leader sequence may e.g. be a 1-15 amino acid (e.g. a 1-10 amino acid) peptide, e.g. AQ, AQGT, AQVDAKFD, AQGTVDAKFD or AQVDNKFN. Hence, a typical structure of a ligand may e.g. be Leader - (Domain-Linker)ₙ₋₁ - Domain - Coupling moiety, n may e.g. be 1-7, such as 1, 2, 3, 4, 5, 6 or 7.

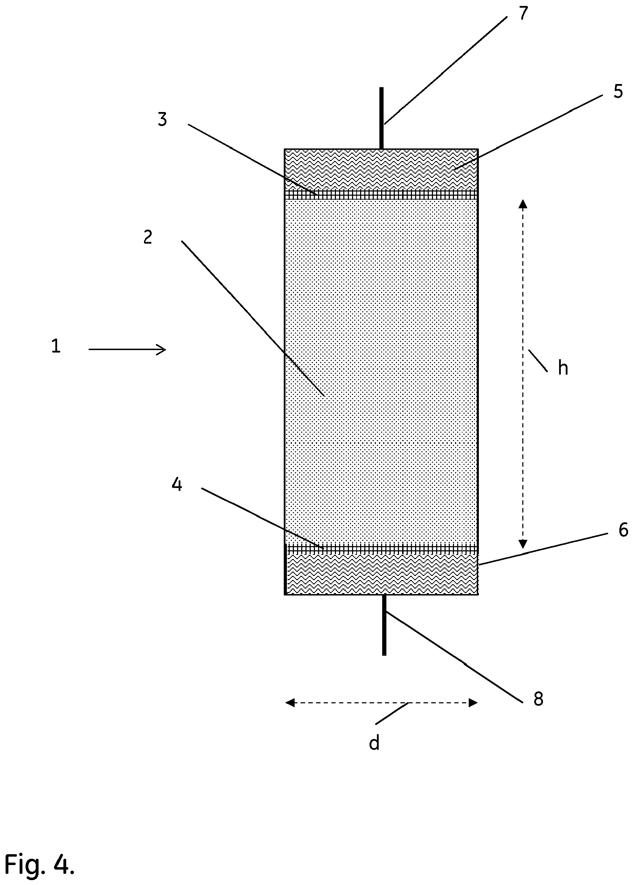

In a second aspect, illustrated by Fig. 4, the invention discloses a chromatography column 1 comprising the separation matrix as described above. The chromatography column can e.g. be an axial packed bed column **1**, where a cylindrical packed bed **2** of matrix particles is confined between two nets/frits **3**,**4** and two distributor structures **5**,**6**, allowing flow of a feed through an inlet **7**, an inlet distributor **5** and an inlet net/frit **3** through the packed bed **2** and then through an outlet frit/net **4**, an outlet distributor **6** and an outlet **8**. The height ***h*** of the packed bed may e.g. be up to 5 cm or up to 10 cm, such as 2-5 cm or 2-4 cm. The diameter ***d*** of the packed bed may e.g. be at least 1 cm, such as at least 1.5 cm or 1.5 - 200 cm, 1.5 - 100 cm, 1.5 - 50 cm or 1.5 - 30 cm.

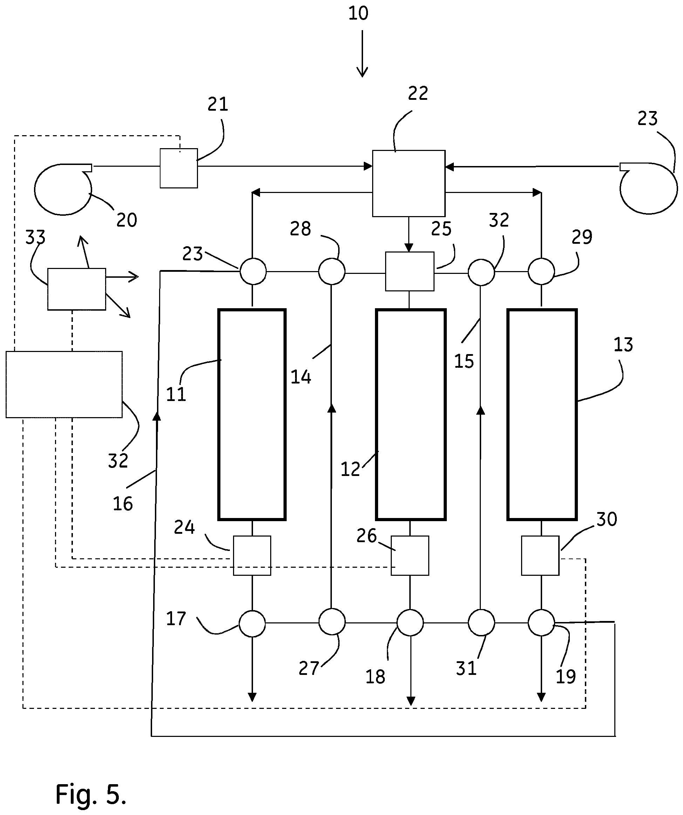

In a third aspect, illustrated by Fig. 5, the invention discloses a chromatography system **10** comprising a plurality of chromatography columns **11,12,13** as disclosed above. The system can suitably be arranged for performing continuous chromatography. It may e.g. comprise at least two, such as at least three chromatography columns **11,12,13** as disclosed above, packed with the same separation matrix and connected with one or more connecting lines **14,15,16** such that liquid can flow from one column **11,12** to a subsequent one **12,13** and from a last column **13** to a first column **11** and each connecting line between two columns may comprise at least one on/off valve **17,18,19,** which may be three-way or four-way valves. The system may further comprise a feed pump **20,** e.g. connected via a first detector **21** to a first valve block **22.** A buffer pump **23** may also be connected to this first valve block **22.** The first valve block **22** can further be connected to the inlet of a first column **11** via a first valve **23.** An outlet end of the first column **11** may be connected to a second valve **17** through a second detector **24.** The first valve block **22** can further be connected to the inlet of a second column **12** via a second valve or valve block **25**. An outlet end of the second column **12** can be connected to valve **18** via a third detector **26**. Furthermore, a valve **27** can be connected between valve **17** and valve **18**. Valve **27** can also be connected to a valve **28** which is also connected to valve **23** and the second valve block **25**. Hereby the effluent from the first column **11** can be directed to the inlet of the second column **12** through connecting line **14**, valves **17, 27, 28** and **25**. Furthermore the first valve block **22** can be connected to the inlet of a third column **13** via valve **29**. An outlet end of the third column **13** may be connected to valve **19** via a fourth detector **30**. Furthermore valve **31** can be connected between valve **18** and valve **19**. Valve **31** can also be connected to valve **32** which may also connected to the second valve block **25** and valve **29**. Hereby the effluent from the second column **12** can be directed to the inlet of the third column **13** through connecting line **15**. The effluent from the third column **13** can be directed to the inlet of the first column **11** through connecting line **16** via valves **19** and **23**. Furthermore, the first, second, third and fourth detectors **21, 24, 26, 30** may all be connected to a determining unit **32**. The determining unit can be adapted to use the detected signals from the detectors to determine breakthrough and saturation points for the three different columns. The determining unit **32** and all the valve blocks, valves and pumps may further be connected to a control unit **33** (all the connections are not shown in the Figure) which is adapted to control the chromatography system in terms of when to remove or add columns from/into the loading zone, change flow rates, start new wash steps, etc. The detectors **21, 24, 26, 30** can e.g. be UV detectors. The control unit **33** may be configured to control the system according to breakthrough data obtained from the determining unit **32**. Alternatively, control unit **33** can use fixed predetermined step times for the switching operations.

In a fourth aspect, the invention discloses a method of separation of antibodies by affinity chromatography. This method comprises the steps of:
a) conveying a process feed through at least a first chromatography column as disclosed above, to adsorb antibodies from the feed. The process feed may e.g. comprise at least 4 mg/ml antibodies, such as 4-15, 4-10, or 4-5 mg/ml and/or the residence time in this step may e.g. be less than 2 min, such as 0.3-1 min or 0.3-0,8 min.;
b) optionally washing the first chromatography column;
c) conveying an eluent through the first chromatography column to elute antibodies; and
d) recovering the eluent with antibodies.
The method can suitably be carried out in the chromatography system **10** disclosed above.

In certain embodiments of the method, in step a) an effluent from the first chromatography column **11** is passed through a second chromatography column **12** packed with the same separation matrix as the first column;
after step a), in a step a'), the process feed is redirected to the second chromatography column **12** and an effluent from the second chromatography column is passed through a third chromatography column **13** packed with the same separation matrix as the first and second columns;
after step a'), in a step a"), the process feed is redirected to the third chromatography column **13** and an effluent from the third chromatography column is passed through the first chromatography column **11**;
step c) is performed before step a'');
after step a'), in a step c'), the eluent is conveyed through the second chromatography column **12** to elute antibodies;
after step a"), in a step c"), the eluent is conveyed through the third chromatography column **13** to elute antibodies; and
the sequence of steps a), a'), a''), c), c') and c'') is optionally repeated one or more times.
The residence time in steps a), a') and a") may e.g. be less than 2 min, such as 0.3-1 min or 0.3-0,8 min.

The method may further, after steps c), c') and c") respectively, comprise steps e), e') and e"), each comprising conveying a cleaning liquid through said first, second and third chromatography columns respectively. The cleaning liquid can be an aqueous alkali solution comprising at least 0.1M (e.g. 0.1- 1M or 0.1 - 0.5 M) alkali. The alkali may e.g be NaOH, but can also be e.g. KOH. The cleaning (also called cleaning in place - CIP) step ensures that any residual feed components are removed from the columns before repetition of the binding and elution steps. Suitable, the ligands are capable of withstanding repeated alkali treatments, e.g. as discussed above where the matrix retains at least 95% of its original IgG-binding capacity after 5 h incubation with 0.5 M NaOH.

After steps e), e') and e") respectively, the method may also comprise equilibration steps f), f) and f'') to reequlibrate the columns for steps a), a') and a") respectively.

### EXAMPLES

### Example 1

Columns: Three HiTrap 5 mL plastic columns (internal diameter 7.0 mm) packed with highly crosslinked spherical agarose beads to a bed height of 3.0 cm. The beads contained 11 mg/ml SpA variant ligands (tetramers of Zvar), covalently coupled via a C-terminal cysteine to high rigidity (crosslinked according to the procedure described in US6602990) agarose beads of 52 micrometers volume-weighted median diameter (d50,v), having a porosity corresponding to a K_{D} value of 0.66 for dextran of Mw 110 kDa.

Feed: Clarified CHO cell supernatant containing 4.0 g/L of a monoclonal IgG antibody, filtered through a 0.22 micrometer filter. 752 g feed was mixed with 1253 g PBS buffer pH 7.4 to give a mAb concentration of 1.5 g/L before loading on the columns. The UV absorbance (300 nm) of this mixture was 695 mAu.

Chromatography: The columns were mounted in an ÄKTA™ PCC (GE Healthcare BioSciences AB, Sweden) system with flowpaths similar to Fig. 5 and the diluted feed was continuously captured on the columns using a 3-column PCC method under the conditions as described in Table 1. Buffers: Equilibration 10mM Phosphate 27mM KCl 140mM NaCl pH 7.4, Wash 1 10mM Phosphate 27mM KCl 140mM NaClpH 7.4, Wash 2 50mM Acetate buffer pH 6, Elution 50mM Acetate buffer pH 3.5, CIP 100mM NaOH. The system was run with predetermined fixed step times.

**Table 1. PCC steps of Example 1.**

| **Step** | **Column volumes (CV)** | **Flow rate (ml/min)** | **Residence time (RT) (min)** | **Step duration time (min)** |
|---|---|---|---|---|
| Load | 28.9 (32.3 CV first load) | 10 | 0.5 | 14.5 (16.2 first load) |
| Wash 1 | 2 | 10 | 0.5 | 1 |
| Wash 2 | 1 | 10 | 0.5 | 0.5 |
| Elution | 3 | 5.0 | 1 | 3 |
| Cleaning in place (CIP) | 1 | 1.0 | 5 | 5 |
| Equilibration | 5 | 10 | 0.5 | 2.5 |

The column turn-around time, including pump washes, was 14.5 min. The mAb concentration in the eluate was determined by measuring the 280 nm UV absorbance in cuvettes and calculating from a predetermined calibration curve.

Chromatograms from the experiment are shown in Fig. 4. The quantified results are shown in Table 2.

**Table 2. Results from Example 1.**

| **Load #** | **Loaded volume (mL)** | **mAb loaded (mg)** | **Eluate (g)** | **mAb concentration (mg/ml)** | **mAb in eluate (mg)** | **Yield (%)** |
|---|---|---|---|---|---|---|
| 1 | 161.5 | 242 | 8.9 | 23.2 | 206 | 85.2* |
| 2 | 144.5 | 217 | 8.9 | 23.7 | 211 | 97.5* |
| 3 | 144.3 | 216 | 9.2 | 23.7 | 218 | 100.6 |
| 4 | 144.3 | 216 | 9.2 | 23.7 | 218 | 100.7 |
| 5 | 144.4 | 217 | 9.0 | 24.5 | 221 | 101.8 |
| 6 | 144.3 | 216 | 9.7 | 22.9 | 222 | 102.5 |
| 7 | 144.4 | 217 | 9.2 | 24.3 | 224 | 103.4 |
| 8 | 144.5 | 217 | 9.1 | 24.7 | 224 | 103.6 |
| 9 | 144.4 | 217 | 9.6 | 23.2 | 223 | 102.8 |
| 10 | 144.4 | 217 | 9.2 | 24.3 | 224 | 103.4 |
| 11 | 144.4 | 217 | 9.0 | 24.5 | 221 | 101.8 |
| 12 | 144.2 | 216 | 9.3 | 24.3 | 226 | 104.6 |

| | | | | | | |
|---|---|---|---|---|---|---|
| *Before reaching steady state. | | | | | | |

At steady state, the dynamic capacity was on the average 43 g/L, at 45% breakthrough and 0.5 min residence time. The productivity, calculated as mAb concentration/(residence time * number of columns), with the residence time in h, was 60 g/L h.

### Example 2

This 3-column PCC experiment was run with the undiluted 4.0 mg/L supernatant of Example 1 as the feed. The residence time during loading was 2.5 min and the conditions as listed in Table 3. In this experiment, the UV absorption after each column was measured and used to automatically switch columns at 5% breakthrough.

**Table 3. PCC steps of Example 2.**

| **Step** | **Buffer** | **Column volumes** | **Residence Time (min)** |
|---|---|---|---|
| Equilibration | 10mM Phosphate 27mM KCl 140mM NaCl pH 7.4 | 5.5 | 1.5 |
| Feed | 4 mg/mL mAb5 fed batch (0.22 µm) | 5% BT | 2.5 |
| Wash 1 | 10mM Phosphate 27mM KCl 140mM NaCl pH 7.4 | 2 | 2 |
| Wash 2 | 50mM Acetate buffer pH 6 | 1.5 | 1.5 |
| Elution | 50mM Acetate buffer pH 3.5 | 3 | 4 |
| CIP | 100mM NaOH | 3 | 5 |
| ReEquilibration | 10mM Phosphate 27mM KCl 140mM NaCl pH 7.4 | 5 | 1.5 |

The average amount of mAb in each column eluate was 270 mg and the dynamic binding capacity was on the average 54 g/L..

### Example 3

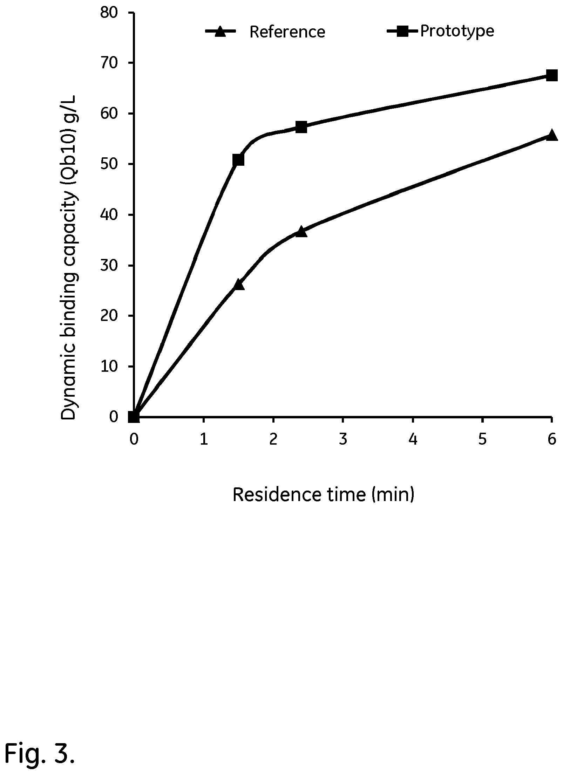

The dynamic binding capacity (10% breakthrough, Qb10) for mAb from the cell supernatant of Example 1 on columns of the same type as in Example 1 was determined as a function of residence time using standard methodology. The measurements were made a) on the same matrix as in Example 1 (Prototype) and b) on a matrix with larger bead size (Reference). In the latter case the matrix contained 10.5 mg/ml SpA variant ligands (tetramers of Zvar), covalently coupled via a C-terminal cysteine to high rigidity (crosslinked according to the procedure described in US6602990) agarose beads of 85 micrometers volume-weighted median diameter (d50,v), having a porosity corresponding to a K_{D} value of 0.69 for dextran of Mw 110 kDa. The results are plotted in Fig. 3 as dynamic binding capacity vs. residence time.

This written description uses examples to disclose the invention, including the best mode, and also to enable any person skilled in the art to practice the invention, including making and using any devices or systems and performing any incorporated methods. The patentable scope of the invention is defined by the claims, and may include other examples that occur to those skilled in the art. Such other examples are intended to be within the scope of the claims if they have structural elements that do not differ from the literal language of the claims, or if they include equivalent structural elements with insubstantial differences from the literal languages of the claims. Any patents or patent applications mentioned in the text are hereby incorporated by reference in their entireties, as if they were individually incorporated.

## Claims

1. A chromatography system arranged for performing continuous chromatography, said system comprising at least two, such as at least three chromatography columns (11,12,13), connected with one or more connecting lines (14,15,16) such that liquid can flow from one column (11,12) to a subsequent one (12,13) and from a last column (13) to a first column (11) and each connecting line (14, 15, 16) between two columns may comprise at least one on/off valve (17,18,19),
wherein each column is packed with a separation matrix comprising porous spherical particles to which antibody-binding protein ligands have been covalently immobilized, wherein the density of said ligands is above 10 mg/ml, and the volume-weighted median diameter of said particles is in the range of 30 - 55 µm; and said porous spherical particles comprise a crosslinked polysaccharide and have a gel phase distribution coefficient, expressed as K_{D} for dextran of molecular weight 110 kDa, of 0.6-0.8.

2. The chromatography system according to claim 1, wherein the at least one on/off valve (17,18,19) is a three-way valve or a four-way valve.

3. The chromatography system according to any of the preceding claims, further comprising a feed pump (20) connected via a first detector (21) to a first valve block (22), and optionally wherein the first valve block (22) is connected to an inlet of a first column (11) via a first valve (23).

4. The chromatography system according to any of the preceding claims, further comprising an outlet end of the first column (11) connected to a second valve (17) through a second detector (24), and/or the first valve block (22) being connected to the inlet of a second column (12) via a second valve or valve block (25).

5. The chromatography system according to any of the preceding claims, further comprising an outlet end of the second column (12) connected to valve (18) via a third detector (26).

6. The chromatography system according to any of the preceding claims, wherein a valve (27) is connected between valve (17) and valve (18), and/or connected to a valve (28) which is also connected to valve (23) and the second valve block (25).

7. The chromatography system according to any of the preceding claims, wherein the effluent from the first column (11) is directed to the inlet of the second column (12) through connecting line (14) and valves (17, 27, 28, 25).

8. The chromatography system according to any of the preceding claims, wherein the first valve block (22) is connected to the inlet of a third column (13) via valve (29), and/or an outlet end of the third column (13) is connected to valve (19) via a fourth detector (30).

9. The chromatography system according to any of the preceding claims, wherein valve (31) is connected between valve (18) and valve (19), and optionally valve (31) is also connected to valve (32) which may also connected to the second valve block (25) and valve (29).

10. The chromatography system according to any of the preceding claims, wherein the effluent from the third column (13) is directed to the inlet of the first column (11) through connecting line (16) via valves (19) and (23).

11. The chromatography system according to any of the preceding claims, wherein the first, second, third and fourth detectors (21, 24, 26,30) are all connected to a determining unit (32).

12. The chromatography system according to claim 14, wherein the determining unit (32) and all the valve blocks (22, 25), valves (17, 18, 19, 23, 27, 28, 29, 31, 32) and pumps (20, 23) are further connected to a control unit (33), said control unit (33) optionally configured to control the system according to breakthrough data obtained from the determining unit (32), or alternatively, said control unit (33) uses fixed predetermined step times for switching operations.

13. A periodic countercurrent chromatography (PCC) column comprising a packed bed (2) of separation matrix comprising porous spherical particles to which antibody-binding protein ligands have been covalently immobilized, wherein the density of said ligands is above 10 mg/ml, and the volume-weighted median diameter of said particles is in the range of 30 - 55 µm; and said porous spherical particles comprise a crosslinked polysaccharide, wherein said packed bed has a bed height (h) of up to 2-5 cm.

14. A method of separation of antibodies by affinity chromatography, wherein the method is carried out with the chromatography system of any one of claims 1-12.

15. The method according to claim 14, wherein the chromatography columns are according to claim 13.
